# EUROPEAN PATENT SPECIFICATION

(11) **EP 2 515 853 B1**
(45) Date of publication and mention of the grant of the patent: **19.03.2014**
(21) Application number: 10799028.5
(22) Date of filing: 22.12.2010
(51) Int. Cl.: A61P 11/06, A61P 11/00, A61K 45/06, A61K 31/167, A61K 31/40, A61K 9/12, A61K 9/00

(54) **Combination therapy for COPD**
Kombinationstherapie für COPD
Polythérapie pour la COPD

(30) Priority: 23.12.2009 EP 09180670
(43) Date of publication of application: 31.10.2012
(73) Proprietor: Chiesi Farmaceutici S.p.A., 43100 Parma (IT)
(72) Inventor: BONELLI, Sauro, I-43100 Parma (IT); ZAMBELLI, Enrico, I-43100 Parma (IT); USBERTI, Francesca, I-43100 Parma (IT)
(74) Representative: Minoja, Fabrizio
(86) International application number: PCT/EP2010/070477
(87) International publication number: WO 2011/076841

(56) References cited:
- EP-A1- 1 894 568
- WO-A1-00/07567
- WO-A1-2008/000482
- WO-A2-2005/074900
- US-A1- 2002 025 299
- US-A1- 2006 257 324
- "Glycopyrronium Bromide" In: "Martindale The complete drug reference", 1 January 2002 (2002-01-01), Pharmaceutical Press, XP002579323, ISBN: 0853694990 vol. 33, pages 467-467, the whole document

## Description

### FIELD OF THE INVENTION

The present invention relates to pharmaceutical aerosol solution formulations intended for use with pressurized metered dose inhalers, comprising glycopyrronium chloride and formoterol or a salt thereof. The invention further relates to use of such formulations in the prevention and therapy of respiratory disorders, including COPD.

### BACKGROUND OF THE INVENTION

Glycopyrronium bromide (also known as glycopyrrolate) is a muscarinic M3 anticholinergic agent used to reduce salivation associated with administration of certain anaesthetics, and as adjunctive therapy for peptic ulcers. It has also been reported to be effective in the treatment of asthmatic symptoms (Hansel et al., Chest 2005; 128:1974-1979).

WO 2005/107873 relates to use of glycopyrrolate for the treatment of childhood asthma.

WO 01/76575 discloses a controlled release formulation for pulmonary delivery of glycopyrrolate. The formulation is intended for use in treatment of respiratory disease, in particular chronic obstructive pulmonary disease (COPD). The application focuses on dry powder formulations suitable for delivery by means of a dry powder inhaler (DPI).

WO 2005/074918 discloses combinations of glycopyrrolate with glucocorticoid drugs, and their use for treating diseases of the respiratory tract.

WO 2005/110402 refers to combinations of glycopyrrolate and a beta-2 agonist of the class of indane or of benzothiazole-2-one derivatives for treatment of inflammatory or obstructive airway diseases.

WO 2006/105401 refers to combinations of an anticholinergic, a corticosteroid and a long-acting beta agonist for prevention and treatment of respiratory, inflammatory or obstructive airway diseases. The anticholinergic is optionally glycopyrrolate.

According to WO 2007/057223 and WO 2007/057222, combinations of glycopyrronium bromide respectively with an anti-inflammatory steroid and, in particular, with mometasone furoate provide a therapeutic benefit in the treatment of inflammatory and obstructive airways diseases.

WO 2007/057221 and WO 2007/057219 refer to combinations of a glycopyrronium salt with an indanyl derivative beta-2 agonist (or analogue) and respectively with an anti-inflammatory steroid and, in particular, with mometasone furoate.

Other counterions (including *inter alia* the chloride ion) have been mentioned as possible alternatives to the bromide counterion of glycopyrronium. WO 2006/100453 proposes the use of the iodide, acetate and sulphate salts as an alternative to glycopyrronium bromide due to milling difficulties associated with the latter.

WO 00/07567 A1 in Example 4 discloses a medicinal aerosol formulation (suspension) wherein to a micronized formoterol fumarate, micronized glycopyrronium **bromide** and micronized disodium cromoglycate mixture, is added a propellant mixture of HFA, dinitrogen monoxide, and a 2% by weight of ethanol.

Martindale Jan. 2002 monograph on glycopyrronium **bromide** shows that in investigations on compatibility of this substance with infusion solutions for injections and additives showed that the stability of glycopyrronium bromide is questionable above a pH of 6 owing to ester hydrolysis.

US 2002/025299 A1 discloses pressurised aerosol solution formulations of different active ingredients among which formoterol or its combination with beclometasone dipropionate acidified by HCl stored in particular kind of cans.

WO 2005/074900 A2 discloses an inhalable combination of an anticholinergic and a beta-2 minetic for the treatment of inflammatory or obstructive respiratory diseases, such as COPD. In the description racemic glycopyrrolate, one of its enantiomers (R,R-glycopyrrolate) or diastereoisomers and salts are cited in general, however, in the Examples only the DPI formulation and the pMDI suspension formulation of the R,R-enantiomer in combination with formoterol are disclosed.

WO 2008/000482 A1 discloses a process for preparing dry powder formulations of a glycopyrronium salt with an antiadherent agent (metal stearate and/or crystalline sugar) and admixing carrier particles.

EP 1 894 568 A1 discloses a medicament comprising a glycopyrronium salt, a beta-2 agonist (such as formoterol) and a corticosteroid for simultaneous, sequential or separate administration in treatment of an inflammarory or obstructive airways disease. The medicament, in inhalable form, may be an aerosol solution or dispersion (suspension) in a propellant, a nebulizable aqueous composition, a dry powder (DPI) composition. The Examples are directed to DPI formulations or only to dispersions of the micronised active ingredients in HFA ethanol mixtures in presence of lactose and/or oleic acid, if required.

US 2006/0257324 discloses the delivery of a combination of two or more dissolved drugs in a HFA propellant-cosolvent system with substantially the same particle size distribution allowing their co-deposition in the same lung region tract. The formulation comprises a beta-2agonist (formoterol or carmoterol exemplified) and a corticosteroid (beclometasone dipropionate exemplified), or an anticholinegic such as ipratropium, oxitropium, tiotropium or glycopyrronium bromide (only generically cited).

Until the present disclosure there was no published evidence that glycopyrronium chloride is either clinically effective or capable of being formulated in a manner suitable for administration to patients with respiratory disease. The present inventors have observed that glycopyrronium chloride has several advantages over glycopyrronium bromide with respect to pharmaceutical formulations. In particular, glycopyrronium chloride has better solubility properties than glycopyrronium bromide, and it has also been found to have better compatibility with other active ingredients, especially with formoterol.

Formoterol is a beta-2 agonist drug capable of relaxing smooth muscle in the bronchi and opening the airways to reduce wheezing conditions. It is commonly used in the management of asthma and other respiratory conditions.

Recently an effective combination therapy comprising formoterol fumarate and beclometasone dipropionate (a corticosteroid) has become available under the trade-name Foster^{®}. Foster^{®} is designed for delivery by aerosol to the lungs using a pressurized metered dose inhaler (pMDI). It has long been known that aerosol solutions of formoterol fumarate are relatively unstable and have a short shelf-life when stored under suboptimal conditions. The Foster^{®} formulation incorporates a quantity of inorganic acid in order to stabilize the formoterol component (as described in EP 1157689).

It would be desirable to provide a clinically useful combination aerosol product that combines the therapeutic benefits of formoterol and glycopyrronium chloride, optionally in conjunction with beclometasone dipropionate. Such a product would need to be formulated in a manner such that each individual pharmaceutically active component is delivered to the lungs in effective and consistent doses over an extended product lifetime, and ideally without the need for storage under special conditions of temperature or humidity.

### SUMMARY OF THE INVENTION

The present invention provides pharmaceutical aerosol formulation comprising:
(a) glycopyrronium chloride at a dosage in the range of 0.5-100 µg per activation; and
(b) formoterol or a salt thereof at a dosage in the range of 1-25 µg per activation;
dissolved in HFA propellant and a co-solvent, characterized in that said composition contains an amount of 1M HCl in the range 0.1-0.3 µg/µl. Optionally the formulation further comprises beclometasone dipropionate.

In another aspect the invention provides the use of a combination product comprising glycopyrronium chloride and formoterol or a salt thereof for the prevention or treatment of COPD and other respiratory diseases.

In yet another aspect, the invention provides a canister for use with a pMDI comprising:
(a) glycopyrronium chloride at a dosage in the range of 0.5-100 µg per activation; and
(b) formoterol or a salt thereof at a dosage in the range of 1-25 µg per activation;
dissolved in a mixture of HFA propellant and a co-solvent characterized in that said composition contains an amount of 1M HCl in the range 0.1-0.3 µg/µl.

### DETAILED DESCRIPTION OF THE PREFERRED EMBODIMENTS

When attempts were made to formulate a combination solution formulation product comprising both glycopyrronium chloride and formoterol it was surprisingly found that the formoterol component underwent significant degradation upon storage under conditions of high temperature and high relative humidity, to an extent that made the product clinically and commercially non-viable. This was despite the presence of acid in the formulation, which would normally be adequate to stabilise the formoterol component.

It also emerged that glycopyrronium chloride is normally unstable in aerosol solution formulations based on HFA and co-solvent, but is stabilized by the inclusion of acid in the formulation.

Upon further analysis it was shown that in the presence of glycopyrronium chloride a proportion of the formoterol component undergoes degradation to a range of different products. Under suboptimal conditions the amounts of these degradation products can exceed the identification and qualification reporting thresholds for new drug products (as defined in ICH Guideline Q3B(R2)). Thus, it became clear that the formulation needed to be altered so as to improve formoterol stability and reduce the levels of unwanted degradation products.

Subsequent experimentation has revealed that one successful approach to avoiding these stability issues is the inclusion of an optimised amount of acid in the formulation so that both the formoterol and the glycopyrronium chloride components are stabilized. In particular, the present inventors found that inclusion of an amount of 1M HCl in the range of 0.1-0.3 µg/µl, preferably 0.15-0.28 µg/µl, more preferably 0.18-0.26 µg/µl, even more preferably 0.20-0.23 µg/µl in the solution is sufficient to favour stabilisation of glycopyrronium chloride and formoterol over an extended period of non-optimal storage, thereby ensuring a consistent dose of glycopyrronium chloride and of formoterol for every actuation of the pMDI containing the solution formulation. The amount of acid included in the formulation is conveniently defined in terms of amount of added acid rather than in terms of resulting pH because the latter is poorly defined in non-aqueous systems such as propellant-based solutions.

A further significant discovery is that removal of oxygen from the canister headspace further stabilizes formoterol at all tested concentrations of 1M HCl.

Glycopyrronium chloride, chemically defined as 3-[(cyclopentylhydroxyphenylacetyl)oxy]-1,1-dimethylpyrrolidinium chloride, has two chiral centres corresponding to four potential different stereoisomers with configurations (3R,2'R)-, (3S,2'R)-, (3R,2'S)- and (3S,2'S)-. Glycopyrronium chloride in the form of any of these pure enantiomers or diastereomers or any combination thereof may be used in practising the present invention. In one embodiment of the invention the (3S,2'R),(3R,2'S)-3-[(cyclopentylhydroxyphenylacetyl)oxy]-1,1-dimethylpyrrolidinium chloride racemic mixture is preferred. Glycopyrronium chloride is present in the formulation in an amount in the range from 0.005 to 0.83% (w/w), preferably from 0.010 to 0.13% (w/w), more preferably from 0.015 to 0.04% (w/w), wherein % (w/w) means the amount by weight of the component expressed as percent with respect to the total weight of the composition.

Glycopyrronium chloride can be prepared using any suitable synthesis technique, such as that described in a co-pending application filed by Chiesi Farmaceutici SpA.

The propellant component of the composition may be any pressure-liquefied propellant and is preferably a hydrofluoroalkane (HFA) or a mixture of different HFAs more preferably selected from the group consisting of HFA 134a (1,1,1,2-tetrafluoroethane), HFA 227 (1,1,1,2,3,3,3-heptafluoropropane), and mixtures thereof. The preferred HFA is HFA134a (1,1,1,2-tetrafluoroethane). HFAs may be present in the formulation in an amount in the range from 75 to 95% (w/w), preferably from 85 to 90% (w/w).

The formoterol component of the formulation can be in the form of the free base, or as a salt or a solvate. Preferably the formoterol is provided in the form of formoterol fumarate. Formoterol fumarate can, for instance, be employed in the formulation in an amount of 0.005-0.07% w/w, preferably 0.01-0.02% w/w.

The co-solvent incorporated into formulation of the invention has a higher polarity than that of the propellant and may include one or more substances such as a pharmaceutically acceptable alcohol, in particular ethanol, or a polyol such as propylene glycol or polyethylene glycol.

Advantageously the co-solvent is selected from the group of lower branched or linear alkyl (C₁-C₄) alcohols such as ethanol and isopropyl alcohol. Preferably the co-solvent is ethanol.

The concentration of the co-solvent will vary depending on the final concentration of the active ingredient in the formulation and on the type of propellant. For example ethanol may be used in a concentration comprised in the range from 5 to 25% (w/w), preferably from 8 to 20% (w/w), more preferably from 10 to 15% (w/w). In one of the preferred embodiments the concentration of ethanol is 12% (w/w).

The ratio of propellant to co-solvent in the formulation is in the range 50:50 to 95:5 (w/w).

It is preferred that the pharmaceutically active components of the composition are substantially completely and homogeneously dissolved in the mixture of propellant and co-solvent, i.e. the composition is preferably a solution formulation.

Optionally the solution formulation compositions may comprise other known pharmaceutical excipients or additives. In particular, the compositions of the invention may comprise one or more low volatility components. Low volatility components are useful in order to increase the mass median aerodynamic diameter (MMAD) of the aerosol particles upon actuation of the inhaler and/or to improve the solubility of the active ingredient in the propellant/co-solvent mixture.

The low volatility component, when present, has a vapour pressure at 25°C lower than 0.1 kPa, preferably lower than 0.05 kPa. Examples of low-volatility components are esters such as isopropyl myristate, ascorbyl myristate, tocopherol esters; glycols such as propylene glycol, polyethylene glycol, glycerol; and surface active agents such as saturated organic carboxylic acids (e.g. lauric, myristic, stearic acid) or unsaturated carboxylic acids (e.g. oleic or ascorbic acid).

The amount of low volatility component may vary from 0.1 to 10% w/w, preferably from 0.5 to 5% (w/w), more preferably between 1 and 2% (w/w).

In another embodiment an amount of water comprised between 0.005 and 0.3% (w/w) may optionally be added to the formulations in order to favourably affect the solubility of the active ingredient without increasing the MMAD of the aerosol droplets upon actuation.

Advantageously, the formulations of the invention are free of excipients (such as surfactants) other than the co-solvent, the propellant and a stabilizing amount of an acid.

The pharmaceutical compositions of the invention may further comprise other, additional pharmaceutically active agents for separate, sequential or simultaneous use. Optional additional pharmaceutically active components of the composition include any known compound for the prophylaxis or treatment of respiratory diseases and their symptoms. Examples of these active components are: beta-agonists such as salbutamol, fenoterol, carmoterol (TA-2005), indacaterol, milveterol, vilanterol (GSK642444) terbutaline, salmeterol, bitolterol, and metaproterenol in form of single stereoisomers or mixtures thereof and salts thereof; corticosteroids such as beclometasone dipropionate, fluticasone propionate, butixocort, mometasone furoate, triamcinolone acetonide, budesonide and its 22R-epimer, ciclesonide, flunisolide, loteprednol, and rofleponide; other anti-muscarinic drugs such as methscopolamine, ipratropium bromide, oxitropium bromide and tiotropium bromide; phosphodiesterase IV inhibitors such as: cilomilast, roflumilast, and tetomilast.

In a preferred embodiment, compositions of the invention comprise beclometasone dipropionate (BDP) as active agent in addition to the formoterol and glycopyrronium chloride components. In that embodiment BDP is preferably present in the formulation in an amount of 0.07-0.41% w/w, preferably 0.1-0.3% w/w.

The compositions of the invention can be inhaled from any suitable known pressurized MDI device. Desired doses of the individual pharmaceutically active components of the formulation are dependent on the identity of the component and the type and severity of the disease condition, but are preferably such that a therapeutic amount of the active ingredient is delivered in one or two actuations. Generally speaking, doses of active ingredient are in the range of about 0.5 µg-1000 µg per actuation, e.g. about 1-100 µg/actuation, and sometimes about 5-50 µg/actuation. The skilled person in the field is familiar with how to determine the appropriate dosage for each individual pharmaceutically active ingredient.

With reference to formoterol, the dosage is 1 to 25 µg per actuation, and more preferably about 5 to 15 µg per actuation. In one specific embodiment the dose of formoterol fumarate is about 6 or 12 µg/actuation.

With reference to glycopyrronium chloride, the preferred dosage is about 0.5-100 µg per actuation, preferably about 1-40 µg per actuation, and more preferably about 5-26 µg per actuation. In one specific embodiment the dose of glycopyrronium chloride is about 25 µg/actuation.

With reference to the optional component beclometasone dipropionate, the preferred dosage is about 10 to 2000 µg per actuation, preferably about 20 to 1000 µg per actuation and more preferably about 50-250 µg per actuation. In one specific embodiment the dose of beclometasone dipropionate is about 50, 100, 200 µg/actuation.

The pharmaceutical formulation of the invention is filled into known pMDI devices. Said devices comprise a canister fitted with a metering valve. Actuation of the metering valve allows a small portion of the spray product to be released.

Part or all of the canister may be made of a metal, for example aluminum, aluminum alloy, stainless steel or anodized aluminum. Alternatively the canister may be a plastic can or a plastic-coated glass bottle.

The metal canisters may have part or all of their internal surfaces lined with an inert organic coating. Examples of preferred coatings are epoxy-phenol resins, perfluorinated polymers such as perfluoroalkoxyalkane, perfluoroalkoxyalkylene, perfluoroalkylenes such as poly-tetrafluoroethylene (Teflon), fluorinated-ethylene-propylene (FEP), polyether sulfone (PES) or fluorinated-ethylene-propylene polyether sulfone (FEP-PES) mixtures or combination thereof. Other suitable coatings could be polyamide, polyimide, polyamideimide, polyphenylene sulfide or their combinations.

In certain embodiments canisters having their internal surfaces lined with FEP-PES or Teflon may be used.

In other particular embodiments canisters made of stainless steel may be used.

The container is closed with a metering valve for delivering a daily therapeutically effective dose of the active ingredient. Generally the metering valve assembly comprises a ferrule having an aperture formed therein, a body moulding attached to the ferrule which houses the metering chamber, a stem consisting of a core and a core extension, an inner- and an outer- seal around the metering chamber, a spring around the core, and a gasket to prevent leakage of propellant through the valve.

The gasket seal and the seals around the metering valve may comprise elastomeric material such as EPDM, chlorobutyl rubber, bromobutyl rubber, butyl rubber, or neoprene. EPDM rubbers are particularly preferred. The metering chamber, core and core extension are manufactured using suitable materials such as stainless steel, polyesters (e.g. polybutyleneterephthalate (PBT)), or acetals. The spring is manufactured in stainless steel eventually including titanium. The ferrule may be made of a metal, for example aluminum, aluminum alloy, stainless steel or anodized aluminum. Suitable valves are available from manufacturers such as Valois, Bespak plc and 3M-Neotechnic Ltd.

The pMDI is actuated by a metering valve capable of delivering a volume of between 25-100 µl, preferably between 40-70 µl, and optionally about 50 µl, or about 63 µl per actuation.

Each filled canister is conveniently fitted into a suitable channeling device prior to use to form a metered dose inhaler for administration of the medicament into the lungs of a patient. Suitable channeling devices comprise, for example a valve actuator and a cylindrical or cone-like passage through which medicament may be delivered from the filled canister via the metering valve to the mouth of a patient e.g. a mouthpiece actuator.

In a typical arrangement the valve stem is seated in a nozzle block which has an orifice leading to an expansion chamber. The expansion chamber has an exit orifice which extends into the mouthpiece. Actuator (exit) orifices having a diameter in the range 0.15 - 0.45 mm and a length from 0.30 to 1.7 mm are generally suitable. Preferably, an orifice having a diameter from 0.2 to 0.44 mm is used, e.g. 0.22, 0.25, 0.30, 0.33 or 0.42 mm.

In certain embodiments of the invention, it may be useful to utilize actuator orifices having a diameter ranging from 0.10 to 0.22 mm, in particular from 0.12 to 0.18 nm as those described in WO 03/053501. The use of said fine orifices may also increase the duration of the cloud generation and hence, may facilitate the coordination of the cloud generation with the slow inspiration of the patient.

In case the ingress of water into the formulation is to be avoided, it may be desired to overwrap the MDI product in a flexible package capable of resisting water ingress. It may also be desirable to incorporate a material within the packaging which is able to adsorb any propellant and co-solvent which may leak from the canister (e.g. a molecular sieve).

Optionally the MDI device filled with the formulation of the invention may be utilized together with suitable auxiliary devices favoring the correct use of the inhaler. Said auxiliary devices are commercially available and, depending on their shape and size, are known as "spacers", "reservoirs" or "expansion chambers". Volumatic^{™} is, for instance, one of the most widely known and used reservoirs, while Aerochamber^{™} is one of the most widely used and known spacers. A suitable expansion chamber is reported for example in WO 01/49350.

The formulation of the invention may also be used with common pressurized breath-activated inhalers such as those known with the registered names of Easi-Breathe^{™} and Autohaler^{™}.

The efficacy of an MDI device is a function of the dose deposited at the appropriate site in the lungs. Deposition is affected by the aerodynamic particle size distribution of the formulation which may be characterised in vitro through several parameters.

The aerodynamic particle size distribution of the formulation of the invention may be characterized using a Cascade Impactor according to the procedure described in the European Pharmacopoeia 6th edition, 2009 (6.5), part 2.09.18. An Apparatus E, operating at a flow rate range of 30 1/min to 100 l/min or an Apparatus D -Andersen Cascade Impactor (ACI)-, operating at a flow rate of 28.3 l/min, may be utilized. Deposition of the drug on each ACI plate is determined by high performance liquid chromatography (HPLC).

The following parameters of the particles emitted by a pressurized MDI may be determined:
i) mass median aerodynamic diameter (MMAD) is the diameter around which the mass aerodynamic diameters of the emitted particles are distributed equally;
ii) delivered dose is calculated from the cumulative deposition in the ACI, divided by the number of actuations per experiment;
iii) respirable dose (fine particle dose = FPD) is obtained from the deposition from Stages 3 (S3) to filter (AF) of the ACI, corresponding to particles of diameter ≤ 4.7 microns, divided by the number of actuations per experiment;
iv) respirable fraction (fine particle fraction=FPF) which is the percent ratio between the respirable dose and the delivered dose.
v) "superfine" dose is obtained from the deposition from Stages 6 (S6) to filter, corresponding to particles of diameter ≤ 1.1 microns, divided by the number of actuations per experiment;

The solutions of the invention are capable of providing, upon actuation of the pMDI device in which they are contained, a total FPF higher than 40%, preferably higher than 50%, more preferably higher than 60%.

Moreover the formulations of the invention are capable of providing, on actuation, a fraction higher than or equal to 30% of emitted particles of diameter equal to or less than 1.1 microns as defined by the content stages S6-AF of an Andersen Cascade Impactor, relative to the total fine particle dose collected in the stages S3-AF of the impactor. Preferably the fraction of emitted particles of diameter equal to or less than 1.1 microns is higher than or equal to 40%, more preferably higher than 50%, even more preferably higher than 60%, most preferably higher than 70%.

According to a further aspect of the invention there is provided a method of filling an aerosol inhaler with a composition of the invention. Conventional bulk manufacturing methods and machinery in the field of pharmaceutical aerosol manufacture may be employed for the preparation of large-scale batches for the commercial production of filled canisters.

A first method comprises:
a) preparing a solution of glycopyrronium chloride and formoterol fumarate (and optionally beclometasone dipropionate) in optional co-solvent (e.g. ethanol), mineral acid, propellant comprising a HFA and optionally a low volatility component at a temperature from -50 to -60°C at which the formulation does not vaporize;
b) cold-filling the inhaler with the prepared solution; and
c) placing the valve onto the empty can and crimping.

An alternative method comprises:
a) preparing a solution of glycopyrronium chloride and formoterol fumarate (and optionally beclometasone dipropionate) in a co-solvent (e.g. ethanol), mineral acid, and optionally a low volatility component:
b) filling the open can with the bulk solution;
c) placing the valve onto the can and crimping; and
d) pressure-filling the can with the HFA propellant through the valve.

A further alternative method comprises:
a) preparing a solution of glycopyrronium chloride, formoterol fumarate (and optionally beclometasone dipropionate) and mineral acid in optional co-solvent (e.g. ethanol), optional low volatility component and HFA propellant using a pressurised vessel:
b) placing the valve onto the empty can and crimping; and
c) pressure-filling the can with the final solution formulation through the valve.

In one embodiment of the invention, oxygen is removed from the headspace of the aerosol canister using conventional techniques in order to further stabilize the formoterol component, especially at higher acid concentrations. This can be achieved in different ways depending on the method of filling the container. Purging can be achieved by vacuum crimping or by using propellant, for instance. In a preferred embodiment the second filling method described above is modified to incorporate an oxygen purge into step (c) by vacuum crimping.

The packaged formulations of the invention are stable for extended periods of time when stored under normal conditions of temperature and humidity. In a preferred embodiment the packaged formulations are stable for at least 6 months at 25°C and 60% RH, more preferably for at least 1 year, most preferably for at least 2 years. Stability is assessed by measuring content of residual active ingredient. A "stable" formulation as defined herein means one retaining at least about 85%, preferably at least about 90%, and most preferably at least about 95% of residual content of each active ingredient at a given time point, as measured by HPLC-UV VIS.

The optimized stable formulations meet the specifications required by the ICH Guideline Q1B or CPMP/QWP/122/02 Rev.1 relevant for drug product stability testing for the purposes of drug registration.

The combination product compositions of the invention may be used for prophylactic or therapeutic purposes or for symptomatic relief of a wide range of conditions, and in one aspect the invention therefore relates to use of any of these these pharmaceutical compositions as a medicament. In particular, the combination products of the invention are useful in the prevention or treatment of many respiratory disorders, such as asthma of all types and chronic obstructive pulmonary disease (COPD).

In another aspect the invention relates to a method of preventing or treating a respiratory disease, such as COPD, comprising administering to a patient in need of such treatment a therapeutically effective amount of a pharmaceutical composition according to the invention.

The invention also provides the use of the pharmaceutical compositions of the invention for the therapeutic or palliative treatment or prevention of respiratory diseases and their symptoms.

Respiratory disorders for which use of the pharmaceutical compositions of the invention may also be beneficial are those characterized by obstruction of the peripheral airways as a result of inflammation and presence of mucus, such as chronic obstructive bronchiolitis, chronic bronchitis, emphysema, acute lung injury (ALI), cystic fibrosis, rhinitis, and adult or acute respiratory distress syndrome (ARDS).

### EXAMPLES

### 1) Stability of single, double and triple combination aerosol solution formulations

A study was performed to investigate the stability of a triple combination of formoterol fumarate (FF), glycopyrronium chloride (GLY) and beclometasone dipropionate (BDP) in an aerosol solution formulation, in canister packaging under varied storage conditions:
In addition to the triple combination, the double combinations (FF + BDP; FF + GLY) and the single agent (GLY; were included in the study to evaluate whether any potential interactions between the active ingredients could affect drug stability. GLY as single agent was formulated with and without 1M HCl to evaluate the stabilizing effect of the acid.

The batch compositions are summarised in Table 1:

**Table 1**

| Theoretical unit formulation (µg/actuation for a 63 µl valve) | | | | | | | |
|---|---|---|---|---|---|---|---|
| **Batch description** | **BDP** | **FF** | **GLY** | **Anhydrous ethanol** | **1M HCl** | **HFA 134a** | **Total** |
| FF + GLY | - | 6 | 25 | 8856 | 14 | 64899 | 73800 |
| FF + GLY + BDP | 100 | 6 | 25 | 8856 | 14 | 64799 | 73800 |
| GLY | - | - | 25 | 8856 | - | 64919 | 73800 |
| GLY + acid | - | - | 25 | 8856 | 14 | 64905 | 73800 |
| FF + BDP | 100 | 6 | - | 8856 | 14 | 64824 | 73800 |

Sample batches were stored in an inverted orientation under the following conditions and two canisters were analysed for content at each checkpoint (after 1, 2, and 3 months of storage):
+5°C
+25°C/60% relative humidity (accelerated storage conditions)
+30°C/65% relative humidity
+40°C/75% relative humidity

The residual content of active ingredient was measured using standard chromatographic protocols.

### Results

Regarding the triple combination, BDP and GLY can contents are not significantly affected by time and temperature. In contrast, FF can content is highly dependent on storage conditions: the % residue with respect to time zero decreases with time and temperature.

When the formulation of the triple combination was stored for 3 months at 25°C/60% relative humidity, the respective residual percent amount of the active ingredients versus their corresponding amount at time 0 were determined and reported in the following Table 2:

**Table 2**

| Active ingredient | Residual % amount ± standard deviation | Number of cans (N.) |
|---|---|---|
| FF | 96.6 ± 0.7 | 4 |
| Gly | 96.4 ± 1.3 | 4 |
| BDP | 94.5 ± 0.3 | 4 |

With regard to the double combination of FF + GLY, the GLY component remains stable under all of the tested conditions. As in the triple combination, the formoterol fumarate can content is strongly dependent on time and temperature.

In contrast, the formoterol content in the FF + BDP double combination does not decrease rapidly over time under any of the different storage conditions. These contrasting observations lead to the conclusion that the presence of GLY in combination with FF has the effect of destabilizing the formoterol fumarate.

When the formulation of the double combinations was stored for 3 months at 25°C/60% relative humidity, the respective residual percent amount of the active ingredients versus their corresponding amount at time 0 were determined and reported in the following Table 3:

**Table 3**

| Combination | Active ingredient | Residual % amount ± standard deviation | Number of cans (N.) |
|---|---|---|---|
| FF+Gly | FF | 96.7 ± 0.2 | 4 |
| FF+Gly | Gly | 96.0 ± 0.1 | 4 |
| FF+BDP | FF | 97.0 ± 0.7 | 4 |
| FF+BDP | BDP | 98.9 ± 0.6 | 4 |

The single agent formulation containing GLY is found to maintain a constant content in the presence of 1M HCl, but to be highly dependent on time and temperature of storage if the acid is omitted. See in the following Table 4 the data when the single agent formulation was stored for 3 months at 25°C/60% relative humidity with or without the same amount of acid.

**Table 4**

| Active ingredient | Residual % amount ± standard deviation | Number of cans (N.) |
|---|---|---|
| Gly (no acid) | 90.4 ± 1.2 | 4 |
| Gly (with acid) | 94.4 ± 0.2 | 4 |

### 2) Analysis of impurities/degradation products

All of the formulations preserved at 25°C/60% RH are tested by a standard HPLC/UV VIS method for non-chiral impurities and degradation products of the active components. An MS detector is used to confirm the molecular weights of the detected impurities/degradation products found in the FF+ BDP and FF + GLY + BDP cans.

### Results:

Analyzed by the HPLC/UV method, those formulations comprising both formoterol and GLY have high levels of degradation products related to formoterol fumarate. It is also observed that the amount of each degradation product increases with temperature.

When the formulation of the triple combination was stored for 3 months at 25°C/60% relative humidity, the total percent amount of impurities and/or degradation products expressed versus the initial amount of the respective active ingredient were determined and reported in the following Table 5:

**Table 5**

| Active ingredient | Total impurities % Vs active ingredient | Number of cans (N.) |
|---|---|---|
| FF | 1.1 | 2 |
| Gly | 0.75 | 2 |
| BDP | 0.21 | 2 |

When the formulation of the double combination was stored for 3 months at 25°C/60% relative humidity the total percent amount of impurities and/or degradation products expressed versus the initial amount of active ingredient were determined and reported in the following Table 6:

**Table 6**

| Combination | Active ingredient | Total impurities % Vs active ingredient | Number of cans (N.) |
|---|---|---|---|
| FF+Gly | FF | 1.3 | 2 |
| FF+Gly | Gly | 0.48 | 2 |
| FF+BDP | FF | 0.80 | 2 |
| FF+BDP | BDP | 0.20 | 2 |

The single agent formulation containing GLY is found to maintain a constant content in the presence of 1M HCl, but to be highly dependent on time and temperature of storage if the acid is omitted. See in the following Table 7 the data for the total percent amount of impurities and/or degradation products expressed versus the initial amount of active ingredient when the single agent formulation was stored for 3 months at 40°C/75% relative humidity with or without the same amount of acid.

**Table 7**

| Active ingredient | Total impurities % Vs active ingredient | Number of cans (N.) |
|---|---|---|
| Gly (no acid) | 14.2 | 2 |
| Gly (with acid) | 1.0 | 2 |

### 3) Titration of acid content

Since the stability and impurity test results point to the importance of acid in the formulations to stabilize formoterol fumarate in the presence of glycopyrronium chloride, a series of triple combination formulations is prepared with added 1M HCl varying between 0.191 µg/µl and 0.254 µg/µl. In each test pair of samples, one can has its oxygen removed by vacuum crimping in order to investigate the impact of oxygen on the degradation process.

After 3 months at 25°C/60% RH the samples are analyzed for residual can content of active ingredients and major impurities/degradation products. The GLY and BDP components are stable over the 3 month period and experience little degradation.

Comparing those samples from which oxygen has been removed, a consistent reduction in FF degradation is observed as the acid content is raised from 0.191 µg/µl through to 0.222 and 0.234 µg/µl. The % degradation products at these acid values is below the identification/qualification levels for drug registration.

In summary, based on current results a double or triple combination product comprising glycopyrronium chloride and formoterol fumarate (and optionally beclometasone dipropionate) could be optimally stabilized for clinical and commercial purposes by inclusion of 1M HCl in an amount of between 0.191 and 0.254 µg/µl, preferably between 0.22 and 0.23 µg/µl in a solution formulation that has been purged of oxygen.

## Claims

1. A pharmaceutical composition comprising:
(a) glycopyrronium chloride at a dosage in the range of 0.5-100 µg per actuation; and
(b) formoterol or a salt thereof at a dosage in the range of 1-25µg per actuation; dissolved in an HFA propellant and a co-solvent, **characterised in that** said composition contains an amount of 1M HCl in the range 0.1 - 0.3 µg/µl.

2. A composition according to claim 1 wherein the range of 1M HCl is 0.15 - 0.28 µg/µl.

3. A pharmaceutical composition according to claim 1 or claim 2 wherein the co-solvent is ethanol.

4. A pharmaceutical composition according to any preceding claim further comprising one or more pharmaceutically active ingredients selected from the group consisting of beta-2 agonists, corticosteroids, antimuscarinic agents, and phosphodiesterase (IV) inhibitors.

5. A pharmaceutical composition according to claim 4 wherein the corticosteroid is beclometasone dipropionate.

6. A pharmaceutical composition according to any preceding claim wherein (a) glycopyrronium chloride is at a dosage in the range of 5-26 µg per actuation and (b) formoterol or a salt thereof is at a dosage in the range of 5-15 µg per actuation.

7. A pharmaceutical composition according to any preceding claim wherein (a) glycopyrronium chloride is at a dosage of 25 µg per actuation and (b) formoterol fumarate is at a dosage of 6 or 12 µg per actuation.

8. A pharmaceutical composition according to claim 5 wherein beclometasone dipropionate is at a dosage in the range of 50-250 µg per actuation.

9. A pharmaceutical composition according to any preceding claim which has been substantially purged of oxygen.

10. An aerosol canister comprising the pharmaceutical composition as defined in any preceding claim.

11. A canister according to claim 10 from which the headspace oxygen has been substantially removed.

12. A method of filling the canister as defined in claim 10 or claim 11 comprising the steps of:
(a) preparing a solution of glycopyrronium chloride, formoterol fumarate and optionally beclometasone dipropionate in a co-solvent to which 1M HCl has been added in an amount of 0.19 - 0.25 µg/µl of the final solution;
(b) filling the aerosol canister with said solution;
(c) placing the valve onto the can and (vacuum) crimping; and
(d) pressure-filling the container with HFA propellant through the valve.

13. A kit-of-parts comprising the pharmaceutical composition as defined in any of claims 1 to 9 and further comprising one or more pharmaceutically active ingredients for separate, sequential or simultaneous administration, wherein said pharmaceutically active ingredients are selected from the group consisting of beta agonists, corticosteroids, antimuscarinic agents, and phosphodiesterase (IV) inhibitors.

14. A pharmaceutical composition according to any of claims 1 to 9 for use in the prevention or treatment of asthma and COPD.

## Patentansprüche

1. Pharmazeutische Zusammensetzung, umfassend:
(a) Glycopyrroniumchlorid in einer Dosis im Bereich von 0,5-100 µg pro Sprühstoß und
(b) Formoterol oder ein Salz davon in einer Dosis im Bereich von 1-25 µg pro Sprühstoß,
aufgelöst in einem HFA-Treibmittel und einem Co-Lösungsmittel, **dadurch gekennzeichnet, dass** die Zusammensetzung eine Menge von 1M HCl im Bereich von 0,1-0,3 µg/µl enthält.

2. Zusammensetzung nach Anspruch 1, wobei der Bereich an 1M HCl 0,15-0,28 µg/µl beträgt.

3. Pharmazeutische Zusammensetzung nach Anspruch 1 oder Anspruch 2, wobei das Co-Lösungsmittel Ethanol ist.

4. Pharmazeutische Zusammensetzung nach einem der vorstehenden Ansprüche, des Weiteren umfassend einen oder mehrere pharmazeutisch wirksame Inhaltsstoffe, ausgewählt aus der Gruppe, bestehend aus beta-2-Agonisten, Corticosteroiden, Antimuskarinika und Phosphodiesterase-(IV)-Inhibitoren.

5. Pharmazeutische Zusammensetzung nach Anspruch 4, wobei das Corticosteroid Beclometasondipropionat ist.

6. Pharmazeutische Zusammensetzung nach einem der vorstehenden Ansprüche, wobei (a) Glycopyrroniumchlorid in einer Dosis im Bereich von 5-26 µg pro Sprühstoß vorliegt und (b) Formoterol oder ein Salz davon in einer Dosis im Bereich von 5-15 µg pro Sprühstoß vorliegt.

7. Pharmazeutische Zusammensetzung nach einem der vorstehenden Ansprüche, wobei (a) Glycopyrroniumchlorid in einer Dosis von 25 µg pro Sprühstoß vorliegt und (b) Formoterolfumarat in einer Dosis von 6 oder 12 µg pro Sprühstoß vorliegt.

8. Pharmazeutische Zusammensetzung nach Anspruch 5, wobei Beclometasondipropionat in einer Dosis im Bereich von 50-250 µg pro Sprühstoß vorliegt.

9. Pharmazeutische Zusammensetzung nach einem der vorstehenden Ansprüche, welche im Wesentlichen von Sauerstoff befreit wurde.

10. Aerosolbehälter, umfassend die pharmazeutische Zusammensetzung wie in einem der vorstehenden Ansprüche definiert.

11. Behälter nach Anspruch 10, aus dem der Kopfraumsauerstoff im Wesentlichen entfernt wurde.

12. Verfahren zur Befüllung des Behälters, wie in Anspruch 10 oder 11 definiert, umfassend die Schritte:
(a) Herstellen einer Lösung von Glycopyrroniumchlorid, Formoterolfumarat und gegebenenfalls Beclometasondipropionat in einem Co-Lösungsmittel, zu dem 1M HCl in einer Menge von 0,19-0,25 µg/µl der fertigen Lösung gegeben wurde,
(b) Füllen des Aerosolbehälters mit der Lösung,
(c) Platzieren des Ventils auf dem Behälter und (Vakuum-) Crimpen und
(d) Druckbefüllung des Behälters mit HFA-Treibmittel über das Ventil.

13. Kit, umfassend die pharmazeutische Zusammensetzung, wie in einem der Ansprüche 1 bis 9 definiert, und des Weiteren umfassend einen oder mehrere pharmazeutisch wirksame Inhaltsstoffe für die separate, aufeinanderfolgende oder simultane Verabreichung, wobei der pharmazeutisch wirksame Inhaltsstoff ausgewählt ist aus der Gruppe, bestehend aus beta-Agonisten, Corticosteroiden, Antimuskarinika und Phosphodiesterase-(IV)-Inhibitoren.

14. Pharmazeutische Zusammensetzung nach einem der Ansprüche 1 bis 9 zur Verwendung bei der Vorbeugung oder Behandlung von Asthma und COPD.

## Revendications

1. Composition pharmaceutique comprenant :
a) du chlorure de glycopyrronium à un dosage dans la plage de 0,5-100 µg par actionnement ; et
b) du formotérol ou un sel de celui-ci à un dosage dans la plage de 1-25 µg par actionnement ; dissous dans un agent de propulsion de type HFA et un cosolvant, **caractérisée en ce que** ladite composition contient du HCl à 1 M en une quantité dans la plage de 0,1-0,3 µg/µl.

2. Composition selon la revendication 1, dans laquelle la plage de HCl à 1 M est de 0,15-0,28 µg/µl.

3. Composition pharmaceutique selon la revendication 1 ou la revendication 2, dans laquelle le cosolvant est l'éthanol.

4. Composition pharmaceutique selon l'une quelconque des revendications précédentes, comprenant en outre un ou plusieurs ingrédients pharmaceutiquement actifs choisis dans le groupe constitué par les agonistes bêta-2, les corticostéroïdes, les agents antimuscariniques et les inhibiteurs de phosphodiésterase (IV).

5. Composition pharmaceutique selon la revendication 4, dans laquelle le corticostéroïde est le dipropionate de béclométasone.

6. Composition pharmaceutique selon l'une quelconque des revendications précédentes, dans laquelle (a) le chlorure de glycopyrronium est à un dosage dans la plage de 5-26 µg par actionnement et (b) le formotérol ou un sel de celui-ci est à un dosage dans la plage de 5-15 µg par actionnement.

7. Composition pharmaceutique selon l'une quelconque des revendications précédentes, dans laquelle (a) le chlorure de glycopyrronium est à un dosage de 25 µg par actionnement et (b) le fumarate de formotérol est à un dosage de 6 ou 12 µg par actionnement.

8. Composition pharmaceutique selon la revendication 5, dans laquelle le dipropionate de béclométasone est à un dosage dans la plage de 50-250 µg par actionnement.

9. Composition pharmaceutique selon l'une quelconque des revendications précédentes, qui a été pratiquement débarrassée de l'oxygène.

10. Bombe aérosol, comprenant la composition pharmaceutique telle que définie dans l'une quelconque des revendications précédentes.

11. Bombe selon la revendication 10, dont l'oxygène de l'espace de tête a été pratiquement éliminé.

12. Procédé de remplissage de la bombe telle que définie dans la revendication 10 ou la revendication 11, comprenant les étapes de :
(a) préparation d'une solution de chlorure de glycopyrronium, de fumarate de formotérol et, éventuellement, de dipropionate de béclométasone dans un cosolvant auquel du HCl à 1 M a été ajouté en une quantité de 0,19-0,25 µg/µl de la solution finale ;
(b) remplissage de la bombe aérosol par ladite solution ;
(c) placement de la soupape sur le récipient et sertissage (sous vide) ; et
(d) remplissage sous pression du récipient par un agent de propulsion de type HFA à travers la soupape.

13. Ensemble de pièces comprenant la composition pharmaceutique telle que définie dans l'une quelconque des revendications 1 à 9 et comprenant en outre un ou plusieurs ingrédients pharmaceutiquement actifs pour l'administration séparée, séquentielle ou simultanée, lesdits ingrédients pharmaceutiquement actifs étant choisis dans le groupe constitué par les agonistes bêta, les corticostéroïdes, les agents antimuscariniques et les inhibiteurs de phosphodiésterase (IV).

14. Composition pharmaceutique selon l'une quelconque des revendications 1 à 9, destinée pour l'utilisation dans la prévention ou le traitement de l'asthme ou de la BPCO.
